# EUROPÄISCHE PATENTSCHRIFT

(11) **EP 2 663 278 B1**
(45) Veröffentlichungstag und Bekanntmachung des Hinweises auf die Patenterteilung: **12.04.2017**
(21) Anmeldenummer: 11807901.1
(22) Anmeldetag: 20.12.2011
(51) Int. Cl.: A61K 8/44, A61K 8/81, A61K 8/72, A61Q 19/10, A61Q 5/02

(54) **KOSMETISCHES REINIGUNGSMITTEL**
COSMETIC CLEANING AGENT
PRODUIT D'HYGIÈNE COSMÉTIQUE

(30) Priorität: 20.12.2010 DE 102010063578
(43) Veröffentlichungstag der Anmeldung: 20.11.2013
(73) Patentinhaber: Henkel AG & Co. KGaA, 40589 Düsseldorf (DE)
(72) Erfinder: SARTINGEN, Kirsten, 41379 Brüggen (DE); PETERSOHN, Dirk, 50996 Köln (DE)
(86) Internationale Anmeldenummer: PCT/EP2011/073313
(87) Internationale Veröffentlichungsnummer: WO 2012/084879

(56) Entgegenhaltungen:
- EP-A2- 1 872 771
- EP-A2- 1 886 663
- WO-A2-2012/041831
- DE-A1-102005 002 486
- US-A1- 2006 079 415

## Beschreibung

Die Erfindung liegt auf dem Gebiet der Kosmetik und betrifft Reinigungsmittel, die eine besondere Tensidmischung, ein spezielles kationisches Polymer und ein Trübungs- bzw. ein Perlganzmittel enthalten.

Die Erfindung betrifft weiterhin ein Verfahren zur Herstellung kosmetischer Reinigungsmittel sowie die Verwendung der Reinigungsmittel zur Verbesserung der Hautfeuchtigkeit.

Kosmetische Reinigungsmittel für die Haut und/oder die Haare wie flüssige Seifen, Shampoos, Duschbäder, Schaumbäder sowie Dusch- und Waschgele müssen neben einer guten Reinigungsleistung auch eine gute Haut- und/oder Schleimhautverträglichkeit aufweisen, und auch bei häufiger Anwendung nicht zu starker Entfettung oder Hauttrockenheit führen.

Daher enthalten moderne Hautreinigungsmittel oftmals zusätzliche Pflegekomponenten wie spezielle Polymere, sogenannte Moisturizing-Komponenten und/oder Rückfettungsmittel, um den Feuchtigkeitsgehalt der Haut so wenig wie möglich aus seinem natürlichen Gleichgewicht zu bringen.

Die Einarbeitung und Stabilisierung solcher Pflegekomponenten kann sehr zeitaufwendig und kostspielig werden, insbesondere dann, wenn dafür zusätzliche Stabilisierungsmittel- und/oder - schritte erforderlich sind.

In der Offenlegungsschrift WO2006/076946 werden tensidhaltige Reinigungsmittel zur Regulierung der Hautfeuchtigkeit offenbart, die eine Mischung aus Joghurt-Proteinen und mindestens einem weiteren Wirkstoff, ausgewählt aus der Gruppe der Pflanzenextrakte und/oder der Pflanzenmilche und/oder der Vitamine(derivate), enthalten.

Nachteilig an den - hauptsächlich in den Beispielen der zuvor genannten Offenlegungsschrift offenbarten - Duschbädern ist, dass sie Stabilitätsprobleme aufweisen können.

Insbesondere dann, wenn zur Herstellung von Duschbädern mit reichhaltigerer und cremigerer Textur auf die Verwendung von Joghurt-Proteinen verzichtet werden sollte, trat eine Separation des Opacifiers auf.

Es wurde gefunden, dass in den zuvor genannten Duschbädern das kationische Polymer Polyquaternium-7 gegen kationische Cellulosederivate wie beispielsweise Polyquaternium-10 ausgewechselt werden kann, ohne dass eine Separation des Opacifiers auftritt.

Die Einarbeitung und Stabilisierung kationischer Cellulosederivate in die Duschbäder ist jedoch nachteilig, denn sie erfordert einen warmen Quellvorgang des Polymeren in Wasser als zusätzlichen Herstellungsschritt.

Es besteht weiterhin der Bedarf nach stabilen kosmetischen Reinigungsmitteln mit cremiger Textur und guten Schaumeigenschaften, die auf der behandelten Haut ein angenehmes Hautgefühl hinterlassen.

Der vorliegenden Erfindung lag die Aufgabe zugrunde, stabile trübe bzw. perlglänzende kosmetische Reinigungsmittel mit sehr guter Pflegeleistung und Hautverträglichkeit herzustellen. Insbesondere sollten kosmetische Reinigungsmittel mit reichhaltiger und cremiger Textur hergestellt werden, die den Schutzmantel der Haut nicht aus seinem natürlichen Gleichgewicht bringen.

Die Reinigungsmittel sollten in Kombination mit Wasser einen cremigen, feinporigen Schaum bilden, der sich gut auf der Haut verteilen lässt, und bereits während der Reinigung ein Gefühl der Pflege vermittelt.

Bei der Herstellung der Mittel sollte aus ökologischen Gesichtpunkten (sowie zur Zeitersparnis) soweit wie möglich auf energetisch aufwendige Schritte verzichtet werden.

Gegenstand der Erfindung ist ein kosmetisches Reinigungsmittel, das in einem kosmetischen Träger
(a) ≥ 12 Gew.-% einer Tensidmischung, die
   (i) 8,75 bis 20 Gew.-% mindestens eines anionischen Tensids (i) aus der Gruppe der Alkyl(ether)sulfate, der Ethercarbonsäuren, der Sulfobernsteinsäuremono- und/oder -dialkylester, der Olefinsulfonate und/oder der Alkylsarcoside und
   (ii) 3,25 bis 7,5 Gew.-% mindestens eines unter der INCI-Bezeichnung Cocamidoproylbetain bekannten Tensids (ii) in einem Gewichtsverhältnis (i) : (ii) von (2,65 bis 2,85) : 1 enthält,
(b) mindestens ein kationisches Copolymer, das (Meth)acrylamid und Dialkyldiallylammoniumsalze als Monomere enthält, und
(c) mindestens ein Trübungsmittel und/oder ein Perlganzmittel, enthält,
wobei sich die Mengenangaben auf das Gesamtgewicht des Reinigungsmittels beziehen.

Die erfindungsgemäßen Reinigungsmittel enthalten die Komponenten (a), (b) und (c) in einem kosmetischen Träger, der bevorzugt wässrig oder wässrig-alkoholisch sein kann.

Bevorzugt enthält der kosmetische Träger mindestens 40 Gew.-% Wasser.

Weiterhin kann der kosmetische Träger 0,01 bis 50 Gew.-%, bevorzugt 0,05 bis 45 Gew.-% und insbesondere 0,1 bis 40 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Sorbitol, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Es wurde gefunden, dass erst ab einem Tensidgehalt ((i) + (ii)) von mindestens 12 Gew.-% eine stabile Dispergierung des Perlglanzmittels und/oder Opacifiers c) in einer ein kationisches Polymer (b) enthaltenden Reinigungszusammensetzung erzielt werden kann.

Eine stabile Dispergierung des Perlglanzmittels und/oder Opacifiers c) kann auch bei höheren Tensidgehalten (unter Beibehaltung des Gewichtsverhältnisses (i) : (ii)) erzielt werden.

Allzu hohe Tensidgehalte sollten aber aus Gründen der Hautschonung vermieden werden, deshalb ist es erfindungsgemäß bevorzugt, wenn die Reinigungsmittel maximal 25 Gew.-%, bevorzugt maximal 20 Gew.-% und insbesondere maximal 15 Gew.-% der Tensidmischung (a) enthalten.

Zu den geeigneten anionischen Tensiden (i), die in den erfindungsgemäßen Mitteln eingesetzt werden können, zählen:
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,

Bevorzugte anionische Tenside (i) sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside (i) sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.

Weiterhin besonders bevorzugte anionische Tenside (i) sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Tensid (ii) ist das unter der INCI-Bezeichnung bekannte Tensid Cocamidopropylbetain.

Das Gewichtsverhältnis (i) : (ii) beträgt (2,65 bis 2,85) : 1.

Die Mengangaben beziehen sich auf das Gesamtgewicht des kosmetischen Reinigungsmittels. Die erfindungsgemäßen Reinigungsmittel enthalten mindestens ein kationisches Polymer b), das (Meth)acrylamid und Dialkyldiallylammoniumsalze als Monomere enthält.

Bevorzugt sind kationische Polymere b), die Acrylamid und Dialkyldiallylammoniumsalze wie beispielsweise Halogenid- und/oder Methosulfatsalze enthalten.

Besonders bevorzugt sind kationische Polymere b), die als Monomere Acrylamid und Dialkyldiallylammoniumchlorid enthalten und unter der INCI-Bezeichnung Polyquaternium-7 bekannt sind.

Von den unter der INCI-Bezeichnung Polyquaternium-7 bekannten kationischen Polymeren sind diejenigen besonders bevorzugt, die ein mittleres Molekulargewicht (Gewichtsmittel) von mindestens 500,000, bevorzugt von mindestens 1,000,000 und insbesondere von mindestens 2,000,000 aufweisen.

Geeignete Handelsprodukte, die in den erfindungsgemäßen Reinigungsmitteln eingesetzt werden können, und jeweils eine 8-10%ige wässrige Lösung eines unter der INCI-Bezeichnung bekannten Polymers Polyquaternium-7 enthalten, sind beispielsweise:
Mirapol^{®} 550 (erhältlich von der Firma Rhodia); Merquat^{®} 550, Merquat^{®} 550-L, Merquat^{®} 2200 und Merquat^{®} S (erhältlich von der Firma Ondeo Nalco); Polyquart^{®} 701, Dehyquart^{®} 701 (erhältlich von der Firma Cognis); Conditioner^{®} P7 (erhältlich von der Firma 3V Sigma), Galsilk^{®} 700 (erhältlich von der Firma Galaxy Surfactants) und Tilamar^{®} Quat 740 (erhältlich von der Firma DSM).

Das (die) kationische(n) Polymer(e) b) wird (werden) in den erfindungsgemäßen kosmetischen Reinigungsmitteln - bezogen auf deren Gesamtgewicht - bevorzugt in einer Menge von 0,01 bis 10 Gew.-%, mehr bevorzugt von 0,05 bis 7,5 Gew.-%, besonders bevorzugt von 0,075 bis 5 Gew.-% und insbesondere von 0,1 bis 3 Gew.-% eingesetzt.

Die erfindungsgemäßen Reinigungsmittel enthalten - bezogen auf ihr Gesamtgewicht - bevorzugt 0,01 bis 10 Gew.-% , mehr bevorzugt 0,05 bis 7,5 Gew.-%, besonders bevorzugt 0,1 bis 5 Gew.-% und insbesondere 0,2 bis 3 Gew.-% mindestens eines Trübungsmittels und/oder Perlglanzmittels c).

Geeignete Trübungs- und/oder Perlglanzmittel c) können ausgewählt sein aus der Gruppe
- der Mono- und/oder Diester aus Ethylenglycol, 1,2-Propandiol und/oder Glycerin mit C₈-C₂₄-Fettsäuren,
- der Ester aus Polyethylenglycolen mit C₈-C₂₄-Fettsäuren und/oder
- der Styrol/Acrylat-Copolymere.

Besonders geeignet sind die unter den INCI-Bezeichnungen bekannten Trübungs- und/oder Perlglanzmittel c):
Glycol Distearate, wie beispielsweise das Handelsprodukt Cutina^{®} AGS der Firma Cognis, Glycol Monostearate, wie beispielsweise das Handelsprodukt Cutina^{®} EGMS der Firma Cognis, PEG-3 Distearate, wie beispielsweise das Handelsprodukt Genapol^{®} TS der Firma Clariant, PEG-2 Distearate, wie beispielsweise das Handelsprodukt Kessco^{®} DEGMS der Firma Akzo Nobel, Propylen Glycol Stearate, wie beispielsweise das Handelsprodukt Tegin^{®} P der Firma Goldschmidt und/oder Styrol/Acrylates-Copolymere wie beispielsweise die Handelsprodukte Joncryl^{®} 67 der Firma Johnson Polymers, Suprawal^{®} WS der Firma BASF und/oder Acusol^{®} OP 301 der Firma Rohm & Haas.

Insbesondere geeignet für die Verwendung in den erfindungsgemäßen Reinigungsmitteln sind die unter den INCI-Bezeichnungen bekannten Trübungs- und/oder Perlglanzmittel c):
Glycol Distearate, Glycol Monostearate, PEG-3 Distearate und/oder Styrol/Acrylates-Copolymer.

In einer bevorzugten Ausführungsform enthält ein erfindungsgemäßes kosmetisches Reinigungsmittel bevorzugt
- 8,75 bis 20 Gew.-% mindestens eines anionischen Tensids (i) aus der Gruppe der Alkyl(ether)sulfate, der Ethercarbonsäuren, der Sulfobernsteinsäuremono- und/oder - dialkylester, der Olefinsulfonate und/oder der Acylsarcoside,
- 3,25 bis 7,5 Gew.-% mindestens eines unter der INCI-Bezeichnung Cocamidopropylbetain bekannten Tensids (ii),
   wobei das Gewichtsverhältnis (i) : (ii) (2,65 bis 2,85) : 1 beträgt,
- 0,075 bis 5 Gew.-% mindestens eines kationischen Polymeren, das als Monomere Acrylamid und Dialkyldiallylammoniumsalze wie Halogenid- und/oder Methosulfatsalze enthält, und
- 0,1 bis 5 Gew.-% mindestens eines Perlglanz- und/oder Trübungsmittels aus der Gruppe der unter den INCI-Bezeichnungen bekannten Verbindungen Glycol Distearate, Glycol Monostearate, PEG-3 Distearate und/oder Styrol/Acrylates-Copolymer,
wobei sich die Mengenangaben auf das Gesamtgewicht des kosmetischen Reinigungsmittels beziehen.

Innerhalb dieser Ausführungsform sind kosmetische Reinigungsmittel mehr bevorzugt, die
- 8,75 bis 20 Gew.-% mindestens eines anionischen Tensids (i) aus der Gruppe der Alkyl(ether)sulfate, der Ethercarbonsäuren, der Sulfobernsteinsäuremono- und/oder - dialkylester, der Olefinsulfonate und/oder der Acylsarcoside und
- 3,25 bis 7,5 Gew.-% mindestens eines unter der INCI-Bezeichnung Cocamidopropylbetain bekannten Tensids (ii),
wobei das Gewichtsverhältnis (i) : (ii) (2,65 bis 2,85) : 1 beträgt,
- 0,1 bis 3 Gew.-% mindestens eines unter der INCI-Bezeichnung Polyquaternium-7 bekannten kationischen Polymers und
- 0,2 bis 3 Gew.-% Styrol/Acrylates-Copolymer enthalten,
wobei sich die Mengenangaben auf das Gesamtgewicht des kosmetischen Reinigungsmittels beziehen.

Zur Unterstützung der Hautpflege und/oder zur nachhaltigen Befeuchtung der Haut ist es vorteilhaft, wenn die erfindungsgemäßen kosmetischen Reinigungsmittel zusätzlich mindestens einen die Hautfeuchtigkeit und/oder den natürlichen Säureschutzmantel der Haut positiv beeinflussenden Wirkstoff in einer Menge von 0,001 bis 10 Gew.-%, bevorzugt von 0,005 bis 7,5 Gew.-%, besonders bevorzugt von 0,01 bis 5 Gew.-% und insbesondere von 0,02 bis 4 Gew.-% enthalten, wobei sich die Mengenangaben auf das Gesamtgewicht der Reinigungsmittel beziehen. Geeignete Wirkstoffe, die die Hautfeuchtigkeit und/oder den natürlichen Säureschutzmantel der Haut positiv beeinflussen, und ohne negative Beeinflussung der gewünschten Textur in die Reinigungsmittel eingearbeitet werden können, sind beispielsweise:
a. Harnstoff,
b. Guanidin,
c. Imidazol,
d. Aminosäuren,
e. Milchsäure bzw. Natriumlactat,
f. Glycerin,
g. 1,2- und/oder 1,3 Diole,
h. Taurin,
i. Vitamine,
j. sowie die physiologisch verträglichen Salze und/oder Derivate dieser Wirkstoffe.

Geeignete Aminosäuren können ausgewählt sein aus den essentiellen Aminosäuren Isoleucin, Leucin, Lysin, Methionin, Phenylalanin, Threonin, Thryptophan und/oder Valin, den semiessentiellen Aminosäuren Arginin und/oder Histidin und/oder den nicht-essentiellen Aminosäuren Alanin, Asparaginsäure, Cystein, Cystin, Glutaminsäure, Glycin, Prolin, Serin und/oder Tyrosin.

Besonders geeignete Aminosäuren sind Glycin, L-Arginin, L-Prolin und/oder L-Valin.

Geeignete 1,2- und/oder 1,3 Diole sind beispielsweise 1,2-Propandiol, 1,2-Pentandiol, 1,2-Hexandiol, 1,2-Dodecandiol und/oder 1,3-Propandiol. Besonders geeignet sind 1,2-Propandiol und/oder 1,2-Hexandiol.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäßen Reinigungsmittel können bevorzugt Vitamine, Provitamine und Vitaminvorstufen aus den Gruppen A, B, E und H enthalten.
Insbesondere bevorzugt sind Vitamine, Provitamine und Vitaminvorstufen aus den Gruppen B und/oder H wie Nicotinsäureamid, Biotin, Pantolacton und/oder Panthenol.
In einer weiteren bevorzugten Ausführungsform der Erfindung enthalten die kosmetischen Reinigungsmittel bevorzugt mindestens zwei Wirkstoffe aus der Gruppe Glycerin, Milchsäure und/oder Natriumlactat, Glycin, Taurin, L-Arginin, L-Prolin, L-Valin, Nicotinsäureamid und/oder Panthenol.

In einer bevorzugten Ausführungsform enthält ein erfindungsgemäßes kosmetisches Reinigungsmittel bevorzugt
- 8,75 bis 20 Gew.-% mindestens eines anionischen Tensids (i) aus der Gruppe der Alkyl(ether)sulfate, der Ethercarbonsäuren, der Sulfobernsteinsäuremono- und/oder - dialkylester, der Olefinsulfonate und/oder der Acylsarcoside,
- 3,25 bis 7,5 Gew.-% mindestens eines unter der INCI-Bezeichnung Cocamidopropylbetain bekannten Tensids (ii),
   wobei das Gewichtsverhältnis (i) : (ii) (2,65 bis 2,85) : 1 beträgt,
- 0,075 bis 5 Gew.-% mindestens eines kationischen Polymeren, das als Monomere Acrylamid und Dialkyldiallylammoniumsalze wie Halogenid- und/oder Methosulfatsalze enthält,
- 0,1 bis 5 Gew.-% mindestens eines Perlglanz- und/oder Trübungsmittels aus der Gruppe der unter den INCI-Bezeichnungen bekannten Verbindungen Glycol Distearate, Glycol Monostearate, PEG-3 Distearate und/oder Styrol/Acrylates-Copolymer, und
- 0,005 bis 7,5 Gew.-% mindestens eines die Hautfeuchtigkeit und/oder den natürlichen Säureschutzmantel der Haut positiv beeinflussenden Wirkstoffs, ausgewählt aus Harnstoff, Guanidin, Imidazol, Aminosäuren, Milchsäure bzw. Natriumlactat, Glycerin, 1,2- und/oder 1,3 Diolen, Taurin, Vitaminen und/oder den physiologisch verträglichen Salzen und/oder Derivaten dieser Wirkstoffe,
wobei sich die Mengenangaben auf das Gesamtgewicht des kosmetischen Reinigungsmittels beziehen.
Innerhalb dieser Ausführungsform sind kosmetische Reinigungsmittel mehr bevorzugt, die
- 8,75 bis 20 Gew.-% mindestens eines anionischen Tensids (i) aus der Gruppe der Alkyl(ether)sulfate, der Ethercarbonsäuren, der Sulfobernsteinsäuremono- und/oder - dialkylester, der Olefinsulfonate und/oder der Acylsarcoside,
- 3,25 bis 7,5 Gew.-% mindestens eines unter der INCI-Bezeichnung Cocamidopropylbetain bekannten Tensids (ii),
   wobei das Gewichtsverhältnis (i) : (ii) (2,65 bis 2,85) : 1 beträgt,
- 0,1 bis 3 Gew.-% mindestens eines unter der INCI-Bezeichnung Polyquaternium-7 bekannten kationischen Polymers,
- 0,2 bis 3 Gew.-% Styrol/Acrylates-Copolymer und
- 0,01 bis 5 Gew.-% mindestens eines Wirkstoffs aus der Gruppe Glycerin, Milchsäure und/oder Natriumlactat, Glycin, Taurin, L-Arginin, L-Prolin, L-Valin, Nicotinsäureamid und/oder Panthenol enthalten,
wobei sich die Mengenangaben auf das Gesamtgewicht des kosmetischen Reinigungsmittels beziehen.

Innerhalb dieser Ausführungsform ist es mehr bevorzugt, wenn die Reinigungsmittel mindestens zwei Wirkstoffe aus der Gruppe Glycerin, Milchsäure und/oder Natriumlactat, Glycin, Taurin, L-Arginin, L-Prolin, L-Valin, Nicotinsäureamid und/oder Panthenol enthalten.

Innerhalb dieser Ausführungsform ist es besonders bevorzugt, wenn die Reinigungsmittel mindestens drei Wirkstoffe aus der Gruppe Glycerin, Milchsäure und/oder Natriumlactat, Glycin, Taurin, L-Arginin, L-Prolin, L-Valin, Nicotinsäureamid und/oder Panthenol enthalten.

Innerhalb dieser Ausführungsform ist es insbesondere bevorzugt, wenn die Reinigungsmittel mindestens vier Wirkstoffe aus der Gruppe Glycerin, Milchsäure und/oder Natriumlactat, Glycin, Taurin, L-Arginin, L-Prolin, L-Valin, Nicotinsäureamid und/oder Panthenol enthalten.

Neben den zuvor genannten Wirkstoffen können die erfindungsgemäßen Reinigungsmittel eine Reihe weiterer Wirkstoffe enthalten, die ihnen vorteilhafte Eigenschaften verleihen.

Zu den bevorzugten fakultativen Wirkstoffen, die in den erfindungsgemäßen Reinigungsmitteln eingesetzt werden können, zählen beispielsweise:
- Öl-, Wachs- und/oder Fettkomponenten, die in den Reinigungsmitteln (bezogen auf ihr Gesamtgewicht) bevorzugt in einer Menge von 0,01 - 20 Gew.-%, besonders bevorzugt von 0,05 - 15 Gew.% und insbesondere von 0,1 - 10 Gew.% eingesetzt werden können,
- nichtionische Tenside und/oder nichtionische Emulgatoren, die in den Reinigungsmitteln (bezogen auf ihr Gesamtgewicht) bevorzugt in einer Menge von 0,1 bis 15 Gew.-%, bevorzugt 0,5 bis 12,5 Gew.-% und insbesondere 1 bis 10 Gew.-% eingesetzt werden können,
- kationsche Tenside, die in den Reinigungsmitteln bevorzugt in Mengen von 0,1 bis 10 Gew.-%, bezogen auf das gesamte Mittel, eingesetzt werden können. Mengen von 0,2 bis 7,5 Gew.-% und insbesondere von 0,3 bis 5 Gew.-% sind besonders bevorzugt und/oder
- Proteinhydrolysate, die in den Reinigungsmitteln bevorzugt in Mengen von 0,01 - 10 Gew.-%, bezogen auf das gesamte Mittel, eingesetzt werden können. Mengen von 0,1 bis 5 Gew.-% und insbesondere 0,1 bis 3 Gew.-%, sind besonders bevorzugt.

Geeignete Öl- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen in Betracht.
Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.
Es ist daher erstrebenswert, in den kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als Ölkomponente kann weiterhin ein Dialkylether dienen.
Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.
Besonders bevorzugt ist der Di-n-octylether, der im Handel unter der Bezeichnung Cetiol^{®} OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, lsofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Zu den geeigneten nichtionischen Tensiden/Emulgatoren zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Für den Fall, dass ein nichtionisches Tensid in den Reinigungsmitteln eingesetzt wird, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Weiterhin bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

Geeignete kationische Tenside sind beispielsweise quartäre Ammoniumverbindungen, Esterquats und/oder Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®}, Armocare^{®} und Quartamin^{®} vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid^{®} S 18 im Handel erhältliche Stearamidopropyldimethylamin dar.

Geeignete Proteinhydrolysate sind Produktgemische, die durch sauer, basisch oder enzymatisch katalysierten Abbau von Proteinen (Eiweißen) erhalten werden.
Es können Proteinhydrolysate sowohl pflanzlichen als auch tierischen Ursprungs eingesetzt werden.
Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.
Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.
Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.

Weitere Wirk-, Hilfs- und Zusatzstoffe, die in den erfindungsgemäßen kosmetischen Reinigungsmitteln eingesetzt werden können, sind beispielsweise:
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Cholesterin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konsistenzgeber wie Zuckerester, Polyolester oder Polyolalkylether,
- Konservierungsmittel, wie beispielsweise Natriumbenzoat oder Salicylsäure,
- Viskositätsreglerwie Salze (NaCl).

Die erfindungsgemäßen Reinigungsmittel weisen bevorzugt einen pH-Wert in einem hautschonenden Bereich von 4 bis 6,5, bevorzugt von 4 bis 6 und insbesondere von 4,5 bis 5,5 auf.

Die gewünschte reichhaltige cremige Textur der erfindungsgemäßen kosmetischen Reinigungsmittel lässt sich besonders gut in einem bestimmten Viskositätsbereich der Mittel erzielen.

Erfindungsgemäß bevorzugte kosmetische Reinigungsmittel weisen daher bevorzugt eine Viskosität im Bereich von 5,000 bis 14,000 mPas, bevorzugt von 6,000 bis 13,000 mPas und insbesondere von 7,500 bis 11,000 mPas (jeweils gemessen mit einem Haake Rotationsviskosimeter VT550; 20°C; Meßeinrichtung MV; Spindel MV II; 8 UPM) auf. Zusammensetzungen dieser Viskosität lassen sich bequem und einfach aus einem Behälter auf die Hand oder die Anwendungsoberfläche applizieren, ohne zwischen den Fingern hindurchzulaufen und zu tropfen. Gleichzeitig ist die Viskosität der Zusammensetzung niedrig genug, damit eine zufriedenstellende Verteilung auf der Anwendungsoberfläche unter Zuhilfenahme der Hände gewährleistet ist.

Die erfindungsgemäßen Reinigungsmittel weisen hervorragende Eigenschaften in der Anwendung auf der Haut auf.
Sie reinigen die Haut besonders schonend, sind gut verträglich und verleihen der Haut während und nach der Reinigung Feuchtigkeit. Die Haut fühlt sich während und nach der Reinigung glatt und seidig an.
Die Reinigungsmittel weisen eine cremige Textur auf und bilden einen feinporigen Schaum, der sich gut auf der Haut verteilen lässt.
Die erfindungsgemäßen Reinigungsmittel weisen weiterhin den Vorteil auf, dass sie sich einfach - vorzugsweise durch einfaches Mischen der Komponenten bei Raumtemperatur- herstellen lassen. Eine (warme) Vorquellung des kationischen Polymeren in Wasser ist nicht erforderlich.

Ein zweiter Gegenstand der Erfindung ist ein Verfahren zur Herstellung eines stabilen trüben und/oder perlglänzenden kosmetischen Reinigungsmittels, bei dem in einem ersten Schritt eine Tensidkombination aus
(a) mindestens einem anionischen Tensid und
(b) mindestens einem zwitterionischen und/oder einem amphoteren Tensid in einem ersten vorgelegt, und in einem zweiten Schritt - vorzugsweise bei Raumtemperatur- mit
(c) mindestens einem kationischen Copolymer, das (Meth)acrylamid und Dialkyldiallylammoniumsalze als Monomere enthält, und
(d) mindestens ein Trübungsmittel und/oder ein Perlganzmittel kombiniert wird.

Ein dritter Gegenstand der Erfindung ist die kosmetische Verwendung eines erfindungsgemäßen Mittels zur Reinigung und Pflege der Haut sowie zur Verbesserung der Hautfeuchtigkeit.

### Beispiele:

Es wurden die folgenden Ausführungsbeispiele hergestellt (die Mengenangaben beziehen sich - sofern nicht anders angegeben - auf Gew.-%):

### Duschbäder:

| | **A** | **B** | **C** | **D** | **E** |
|---|---|---|---|---|---|
| **Sodium Laureth Sulfate 70% AS** | 13 | 13 | 13 | 14 | 15 |
| **Cocamidopropyl Betaine 40% AS** | 8,5 | 8,5 | 8,5 | 9,375 | 10 |
| **Plantacare^{®1} 818** | - | - | 1 | 1 | - |
| **Conditioner^{®2} P7** | 1,5 | 1,5 | - | - | - |
| **Polyquart^{®3} 701** | - | - | 1,5 | 1,5 | |
| **Merquat^{®4} 550** | - | - | - | - | 1,5 |
| **Styrol-Acrylsäure Copolymer (40% in Wasser)** | 1 | 1 | 1 | - | - |
| **Glycol Distearate** | - | - | - | 1,5 | - |
| **PEG-3 Distearate** | - | - | - | - | 2 |
| **Panthenol 75%** | 0,01 | 0,02 | 0,2 | 0,1 | 0,05 |
| **Nicotinsäureamid** | 0,1 | 0,15 | - | - | - |
| **Glycerin 99,5%** | 0,86 | 1 | 3 | 1 | - |
| **Milchsäure 80% DAB** | 0,1 | - | 0,15 | - | 0,1 |
| **Natriumlactat 60%** | 0,1 | 0,1 | - | - | - |
| **Glycin** | 0,003 | - | - | - | - |
| **Taurin** | - | 0,006 | - | - | - |
| **L-Arginin** | - | - | 0,008 | - | - |
| **L-Prolin** | - | - | - | 0,006 | - |
| **L-Valin** | - | - | - | - | 0,009 |
| **Gluadin**^{®5} **WQ** | - | 0,01 | 0,01 | - | - |
| **PEG-7 Glyceryl Cocoate** | 0,6 | 0,8 | 0,5 | 0,3 | 0,8 |
| **Sodium Benzoate** | 0,4 | 0,5 | 0,3 | 0,4 | 0,4 |
| **Sodium Salicylate** | 0,23 | 0,25 | 0,2 | 0,23 | 0,23 |
| **Parfum** | q.s. | q.s. | q.s. | q.s. | q.s. |
| **Wasser** | ad 100 | ad 100 | ad 100 | ad 100 | ad 100 |

Die Duschbäder wurden mit NaCl auf die gewünschte Viskosität von 5,000 bis 14,000 mPas, (jeweils gemessen mit einem Haake Rotationsviskosimeter VT550; 20°C; Meßeinrichtung MV; Spindel MV II; 8 UPM) eingestellt.

In den Duschbädern wurden die folgenden Handelsprodukte eingesetzt:
¹ C₈-C₁₄-Alkylpolyglucosid (INCI: COCO-GLUCOSIDE); AS: 51 - 53%; Cognis
² Acrylamid Dimethyldiallylammoniumchlorid Copolymer (INCI: POLYQUATERNIUM-7); 3V Sigma
³ Acrylamid Dimethyldiallylammoniumchlorid Copolymer (INCI: POLYQUATERNIUM-7); Cognis,
⁴ Acrylamid Dimethyldiallylammoniumchlorid Copolymer (INCI: POLYQUATERNIUM-7); Ondeo Nalco
⁵ INCI: LAURDIMONIUM HYDROXYPROPYL HYDROLYZED WHEAT PROTEIN; Cognis

## Patentansprüche

1. Kosmetisches Reinigungsmittel, enthaltend in einem kosmetischen Träger
(a) ≥ 12 Gew.-% einer Tensidmischung, die
(i) 8,75 bis 20 Gew.-% mindestens eines anionischen Tensids (i) aus der Gruppe der Alkyl(ether)sulfate, der Ethercarbonsäuren, der Sulfobernsteinsäuremono- und/oder - dialkylester, der Olefinsulfonate und/oder der Acylsarcoside und
(ii) 3,25 bis 7,5 Gew.-% mindestens eines unter der INCI-Bezeichnung Cocamidopropylbetain bekannten Tensids (ii) in einem Gewichtsverhältnis (i) : (ii) von (2,65 bis 2,85) : 1 enthält,
(b) mindestens ein kationisches Copolymer, das (Meth)acrylamid und Dialkyldiallylammoniumsalze als Monomere enthält, und
(c) mindestens ein Trübungsmittel und/oder ein Perlganzmittel,
wobei sich die Mengenangaben auf das Gesamtgewicht des Reinigungsmittels beziehen.

2. Kosmetisches Reinigungsmittel nach Anspruch 1, **dadurch gekennzeichnet, dass** es - bezogen auf sein Gesamtgewicht - 0,01 bis 10 Gew.-%, bevorzugt 0,05 bis 7,5 Gew.-%, mehr bevorzugt 0,075 bis 5 Gew.-% und insbesondere 0,1 bis 3 Gew.-% des kationischen Polymeren b) enthält.

3. Kosmetisches Reinigungsmittel nach einem der Ansprüche 1 oder 2, **dadurch gekennzeichnet, dass** es als kationisches Polymer b) ein unter der INCI-Bezeichnung "Polyquaternium-7" bekanntes Polymer enthält.

4. Kosmetisches Reinigungsmittel nach einem der Ansprüche 1 bis 3, **dadurch gekennzeichnet, dass** es - bezogen auf sein Gesamtgewicht - 0,01 bis 10 Gew.-%, bevorzugt 0,05 bis 7,5 Gew.-%, mehr bevorzugt 0,1 bis 5 Gew.-% und insbesondere 0,2 bis 3 Gew.-% eines Trübungs- und/oder Perlganzmittels c) aus der Gruppe
- der Mono- und/oder Diester aus Ethylenglycol, 1,2-Propandiol und/oder Glycerin mit C_{B}-C₂₄-Fettsäuren,
- der Ester aus Polyethylenglycolen mit C₈-C₂₄-Fettsäuren und/oder
- der Styrol/Acrylat-Copolymere
enthält.

5. Kosmetisches Reinigungsmittel nach einem der Ansprüche 1 bis 4, **dadurch gekennzeichnet, dass** es - bezogen auf sein Gesamtgewicht - 0,001 bis 10 Gew.-%, bevorzugt mindestens 0,005 bis 7,5 Gew.-%, mehr bevorzugt 0,01 bis 5 Gew.-% und insbesondere 0,02 bis 4 Gew.-% mindestens eines die Hautfeuchtigkeit und/oder den natürlichen Säureschutzmantel der Haut positiv beeinflussenden Wirkstoffs enthält, der ausgewählt ist aus
a. Harnstoff,
b. Guanidin,
c. Imidazol,
d. Aminosäuren,
e. Milchsäure bzw. Natriumlactat,
f. Glycerin,
g. 1,2- und/oder 1,3 Diolen,
h. Taurin,
i. Vitaminen,
h. sowie den physiologisch verträglichen Salzen und/oder Derivaten dieser Wirkstoffe.

6. Kosmetisches Reinigungsmittel nach Anspruch 5, **dadurch gekennzeichnet, dass** es mindestens zwei Wirkstoffe aus der Gruppe Glycerin, Milchsäure und/oder Natriumlactat, Glycin, Taurin, L-Arginin, L-Prolin, L-Valin, Nicotinsäureamid und/oder Panthenol enthält.

7. Kosmetische Verwendung eines Mittels nach einem der Ansprüche 1 bis 6 zur Reinigung und Pflege der Haut sowie zur Verbesserung der Hautfeuchtigkeit.

## Claims

1. A cosmetic cleansing agent containing, in a cosmetic carrier,
(a) ≥ 12 wt.% of a surfactant mixture which contains
(i) from 8.75 to 20 wt.% of at least one anionic surfactant (i) from the group consisting of alkyl (ether) sulfates, ether carboxylic acids, monoalkyl and/or dialkyl esters of sulfosuccinic acid, olefin sulfonates and/or acyl sarcosides; and
(ii) from 3.25 to 7.5 wt.% of at least one surfactant (ii), known by the INCI name of cocamidopropyl betaine, in a weight ratio (i):(ii) of (2.65-2.85):1;
(b) at least one cationic copolymer that contains (meth)acrylamide and dialkyl diallyl ammonium salts as monomers; and
(c) at least one opacifier and/or one pearlizer:
wherein the quantities are based on the total weight of the cleansing agent.

2. The cosmetic cleansing agent according to claim 1, **characterized in that** it contains, based on its total weight, from 0.01 to 10 wt.%, preferably from 0.05 to 7.5 wt.%, more preferably from 0.075 to 5 wt.%, and in particular from 0.1 to 3 wt.% of the cationic polymers b).

3. The cosmetic cleansing agent according to one of claims 1 to 2, **characterized in that** it contains a polymer known by the INCI name of polyquaternium-7 as the cationic polymer b).

4. The cosmetic cleansing agent according to one of claims 1 to 3, **characterized in that** it contains, based on its total weight, from 0.01 to 10 wt.%, preferably from 0.05 to 7.5 wt.%, more preferably from 0.1 to 5 wt.%, and in particular from 0.2 to 3 wt.% of an opacifier and/or pearlizer c) from the group consisting of
- monoesters and/or diesters of ethylene glycol, 1,2-propane diol and/or glycerin having C₈-C₂₄ fatty acids,
- esters of polyethylene glycols having C₈-C₂₄ fatty acids and/or
- styrene/acrylate copolymers.

5. The cosmetic cleansing agent according to one of claims 1 to 4, **characterized in that** it contains, based on its total weight, from 0.001 to 10 wt.%, preferably at least from 0.005 to 7.5 wt.%, more preferably from 0.01 to 5 wt.%, and in particular from 0.02 to 4 wt.% of at least one active substance that positively influences the skin moisture and/or the natural protective acid mantle of the skin, selected from:
a. urea,
b. guanidine,
c. imidazole,
d. amino acids,
e. lactic acid or sodium lactate,
f. glycerin,
g. 1 2-and/or 1,3-diols,
h. taurine,
i. vitamins,
j. and the physiologically acceptable salts and/or derivatives of these active substances.

6. The cosmetic cleansing agent according to claim 5, **characterized in that** it contains at least two active substances from the group comprising glycerin, lactic acid and/or sodium lactate, glycine, taurine, L-arginine. L-proline, L-valine, nicotinamide and/or Panthenol.

7. The cosmetic use of an agent according to one of claims 1 to 6 for cleansing and caring for skin and for improving the moisture of the skin.

## Revendications

1. Nettoyant cosmétique, contenant dans un support cosmétique
(a) ≥ 12% en poids d'un mélange de tensio-actifs, qui contient
(i) de 8,75 à 20% en poids d'au moins un tensioactif anionique (i) du groupe des alkyl(éther)sulfakes, des acides éther-carboxyliques, des mono- et/ou dialkylesters de l'acide sulfosuccinique, des sulfonates d'oléfine et/ou les sarcosides d'acyle et
(ii) de 3,25 à 7,5% en poids d'au moins un tensio-actif (ii) connu sous le nom INCI de Cocamidopropylbetain dans un rapport en poids (i):(ii) de (2,65 à 2,85):1,
(b) au moins un copolymère cationique qui contient comme monomères du (méth)acrylamide et des sels de dialkyldiallylammonium, et
(c) au moins un agent opacifiant et/ou un agent nacrant,
les quantités indiquées étant basées sur le poids total du détergent.

2. Nettoyant cosmétique selon revendication 1, **caractérisé en ce qu'**il contient, sur la base de son poids total, de 0:01 à 10% en poids, de préférence de 0,05 à 7,5% en poids, plus préférablement de 0,075 à 5% en poids et notamment de 0,1 à 3% en poids du polymère cationique b).

3. Nettoyant cosmétique selon l'une des revendications 1 à 2, **caractérisé en ce qu'**il contient comme polymère cationique b) un polymère connu sous le nom INCI de « Polyquaternium-7 ».

4. Nettoyant cosmétique selon l'une des revendications 1 3, **caractérisé en ce qu'**il contient, sur la base de son poids total, de 0,01 à 10% en poids, de préférence de 0,05 à 7,5% en poids, de préférence de 0,1 à 5% en poids et en particulier de 0,2 à 3% en poids d'un agent opacifiant et/ou d'un agent nacrant c) du groupe
- des mono- et/ou diesters de l'éthylène glycol, du 1,2-propanediol et/ou du glycérol avec des acides gras en C₈ à C₂₄,
- des esters de polyéthylène-glycols avec des acides gras en C₆ à C₂₄ et/ou
- des copolymères styrène/acrylate.

5. Nettoyant cosmétique selon l'une des revendications 1 à 4, **caractérisé en ce qu'**il contient, sur la base de son poids total, de 0,001 à 10% en poids, de préférence d'au moins 0,005 à 7,5% en poids, plus préférablement de 0,01 à 5% en poids et notamment de 0,02 à 4% en poids d'au moins une substance active qui influe sur l'hydratation de la peau et/ou l'enveloppe de protection acide naturelle de la peau et qui est choisie parmi
a. l'urée,
b. la guanidine,
c. l'imidazole,
d. les acides aminés,
e. l'acide lactique ou le lactate de sodium,
f. le glycérol,
g. les 1,2-et/ou 1,3-diols,
h. la taurine,
i. vitamines,
j. ainsi que les sels physiologiquement acceptables et/ou des dérivés de ces substances actives

6. Nettoyant cosmétique selon la revendication 5, **caractérisé en ce qu'**il contient au moins deux substances actives choisies dans le groupe constitué du glycérol, de l'acide lactique et/ou du lactate de sodium, de la glycine, de la taurine, de la L-arginine, de la L-proline, de la L-valine, de la nicotinamide et/ou du panthénol.

7. Utilisation cosmétique d'un nettoyant selon l'une quelconque des revendications 1 à 6 pour le nettoyage et le soin de la peau et pour améliorer l'hydratation de la peau.
